# EUROPEAN PATENT APPLICATION

(11) **EP 0 694 284 A1**
(43) Date of publication of application: **31.01.1996**
(21) Application number: 95201978.4
(22) Date of filing: 18.07.1995
(51) Int. Cl.: A61B 5/0215, A61B 5/03

(54) **Catheter for performing measurements in the esophagus and/or stomach at various positions**

(30) Priority: 18.07.1994 NL 9401180
(71) Applicant: Dräger Medical Electronics B.V., NL-5680 GA Best (NL)
(72) Inventor: Abrahams, Wilhelmus Wilhelmina, NL-5081 BK Hilvarenbeek (NL); Nieuwesteeg, Michael Wilhelmus Clemens Maria, NL-2803 CK Gouda (NL); Buijs, Arnold, NL-5627 JA Eindhoven (NL); Steeghs, Gerardus Franciscus Joseph, NL-5662 AG Geldrop (NL)
(74) Representative: de Bruijn, Leendert C.

(57) **Abstract**

Catheter (10,12) for accurately positioning a number of sensors (14,22) at various locations inside a patient's body to measure sensor dependent parameters at said various predetermined locations, which catheter comprises at least a first sensor (22) near the distal end thereof and at least a second sensor (14) at a distance of the first sensor (22), first and second coupling means (24) near the proximal end of the catheter for transferring the signals of said first and second sensors, and first and second signal conductors (23,15) between the respective first and second sensors and coupling means (24). The catheter comprises a first elongated catheter part (10) near the distal end of which the first sensor (22) is mounted and through which the first signal conductors (23) extend to the first coupling means (24), a second elongated catheter part (12) near the distal end of which the second sensor (14) is mounted and through which the second signal conductors (15) extend to the second coupling means (24). The second catheter part (12) comprises at least one lumen through which the first catheter part (10), which is longer than said lumen through the second catheter part, can be inserted from the proximal end thereof, such that the distal end of the first catheter part (10) projects over a predetermined distance outside the distal end of the second catheter part (12).

## Description

The invention relates to a catheter for accurately positioning a number of sensors at various locations inside a patient's body to measure sensor dependent parameters at said various predetermined locations, which catheter comprises at least a first sensor near the distal end of the catheter, and at least a second sensor at a distance of the first sensor, first and second coupling means near the proximal end of the catheter for transferring the signals of said first and second sensors, and first and second signal conductors between the respective first and second sensors and coupling means.

Catheters of this type are known for instance from US-4,921,481, US-5,109,870, and US-4,887,610.

US-4,921,481 discloses a system for positioning and monitoring the location of the distal end of a feeding catheter in a patient's gastrointestinal tract and for selectively controlling a feeding device associated therewith by detecting and processing myoelectric signals developed by sensors mounted at various positions along the length of the feeding catheter.

US-5,109,870 discloses a catheter for measuring motility and peristalsis in tubular organs such as the esophagus. The catheter comprises a number of resistance measuring electrodes attached to the catheter at mutual distances for measuring muscular activity at various different locations.

US-4,887,610 discloses a manometric catheter comprising both a pressure sensor and a myoelectric sensor for measuring simultaneous mechanical and electrical events for instance in the esophagus sphincter.

In all these known catheters the sensors are installed at fixed predetermined positions with a predetermined mutual distance. Not every patient, however, has the same physical build and not in all cases it is possible to perform the desired measurements with such catheters because a correct positioning of the various sensors, each at an optimum measuring location, cannot always be obtained. Therefore, there is need for a catheter assembly comprising a number of sensors of which the mutual position can be varied independently such that before the measurements are carried out the sensors can be located each at its optimum location inside the patient's body.

This requirement is now fulfilled with a catheter of the type mentioned in the first paragraph, in that the catheter comprises:
- a first elongated catheter part near the distal end of which the first sensor is mounted and through which the first signal conductors extend to the first coupling means,
- a second elongated catheter part near the distal end of which the second sensor is mounted and through which the second signal conductors extend to the second coupling means, which second catheter part comprises at least one lumen through which the first catheter part, which is longer than said lumen through the second catheter part, can be inserted from the proximal end thereof, such that the distal end of the first catheter part projects over a predetermined distance outside the distal end of the second catheter part.

Because the first elongated catheter part with the first sensor at or on the distal end thereof is able to move through said lumen in the second elongated catheter part it is possible to adjust the distance between both sensors as needed for the specific application.

In general the catheter will be inserted in the patients body with the first catheter part retracted within the second catheter part. As soon as the second sensor on the second catheter part has reached its optimum operational position the movement of the second catheter part is stopped and the second catheter part is held steady. Therafter the first catheter part is shifted through the lumen in the second catheter part such that it's distal end carrying the first sensor will move further into the patient's body untill also the first sensor has reached it's optimum operational position. Thereafter the measurements are performed without further longitudinal movement of any catheter part.

Preferably the length of the first catheter part is at least equal to the length of the lumen through the second catheter part increased with the maximum distance over which the distal end of this first catheter part should project outside the distal end of the lumen through the second catheter part, i.e. the foreseen maximum distance between the sensors.

With respect to a catheter construction comprising two mutually movable catheter parts the attention is drawn to EP-0,258,690. This European patent application discloses a catheter for performing measurements in the urethra and in the urinary bladder. For that purpose the catheter comprises a balloon for closing off the mouth of the urethra, a pressure sensor at one side of said balloon for measuring the pressure inside the urinary bladder and a number of pressure sensors at the other side of the ballon for measuring a pressure profile along at least part of the length of the urethra. As alternative for using a large number of sensors in the urethtra it is proposed to use one single pressure sensor installed on a tubular second catheter part which surrounds the first catheter part carrying the ballon and the bladder sensor.

During operation of the alternative emboddiement the catheter is inserted in the urethra until the balloon is located at the mouth of the urethra. In that position the balloon is inflated to close off the urinary bladder from the urethra. Thereafter, the pressure inside the urinary bladder is measured by the first pressure sensor and the pressure in the urethra is measured by the second pressure sensor. To obtain a pressure profile along at least part of the length of the urethra during operation the second catheter part is gradually retracted from its initial measuring position so that the second pressure sensor as it were scans the inner wall of the urethra and measures a corresponding pressure profile.

Moving the second outer catheter part during the measurement operation is considered disadvantageous because the urethra wall will react to such movement and therewith disturb the steady pressure profile which has to be measured. Just because of cavity wall reactions in general a moving outer catheter part has to be avoided during measurements.

Furthermore moving the outer catheter part in relation to the inner catheter part presumes a fixed position of the inner catheter part, which in general can only be obtained by applying inflatable balloons or other similar devices, which is not always possible.

The principle of a first catheter part which is able to move through a lumen in a further catheter part cannot only be applied with just two parts, but is also applicable with more than two parts.

In this respect in a preferred embodiment the catheter according to the invention comprises:
- a number of first catheter parts each comprising a sensor near the distal end of the respective first catheter part and each comprising signal conductors between said sensor and coupling means near the proximal end,
- a second catheter part comprising a number of lumen, corresponding with the number of first catheter parts, through each of which one of said first catheter parts can be inserted, whereby the length of each of said first catheter parts is longer than the length of the lumen through which it is inserted such, that after inserting the first catheter part in the respective lumen the distal end of said first catheter part projects over a predetermined distance outside the distal end of the lumen through said second catheter part.

With such an embodiment an arbitrary number of sensors can be manoeuvred each to a desired position via a lumen through the first catheter part. The various lumens through the first catheter part end near the distal end of the first catheter part but may also at least partly end at a predetermined distance of said distal end in the side wall of the first catheter part.

In respect to this embodiment the attention is drawn to a prior art catheter described in DE-2,160,287. This catheter comprises two first catheter parts each comprising a sensor near the distal end thereof and a second catheter part which only functions as shielding element and does not comprise a further sensor for carrying out measurements.

The principle defined in the main claim can be developed further in another way, i.e. by realizing a telescopic configuration in which three or even more catheter parts are mutually movable.

Therefore, another preferred embodiment of a catheter according to the invention comprises a third elongated catheter part, which third catheter part comprises at least one lumen through which the second catheter part, which is longer than said lumen through the third catheter part, can be inserted from the proximal end thereof, such that the distal end of the second catheter part projects over a predetermined distance outside the distal end of said lumen through said third catheter part. This third catheter part could for instance act as a protecting sleeve. On the other hand, however, this third catheter part can be embodied such that at or near the distal end of said third catheter part a third sensor is attached and third signal conductors are extending through the third catheter part to third coupling means at the proximal end of the catheter.

More in general the catheter according to the invention may comprise n elongated catheter parts (n equal to or larger than 2) whereby each m-th elongated catheter part (m at least equal to 2 but at the most equal to n) comprises at least one lumen through which the (m-1)-th catheter part, which is longer than the lumen through the m-th catheter part can be inserted from the proximal end thereof such that the distal end of the (m-1)-th catheter part projects over a predetermined distance outside the distal end of the lumen through said m-th catheter part. Also in this general case it is possible to use one or more of the catheter parts exclusively for instance as protecting sleeve but it is preferred to embody at least part of the n elongated catheter parts such that near the distal end of m-th catheter part an m-th sensor is mounted and m-th signal conductors are extending through said m-th catheter part to m-th coupling means at the proximal end of the catheter.

Within the scope of the invention also catheters are conceivable comprising:
- a number of first catheter parts, each comprising a sensor near the distal end of the respective first catheter part and each comprising signal conductors between said sensor and coupling means near the proximal end, and
- a number of second catheter parts, each comprising a lumen through which a first catheter part can be inserted, whereby the length of said first catheter part is longer than the length of the lumen through which it extends such that after inserting the first catheter part in the respective lumen the distal end of the first catheter part projects over a predetermined distance outside the distal end of the lumen through said second catheter part,
- a third catheter part, comprising a number of lumen, corresponding to the number of second catheter parts, through each of which one of said second catheter parts can be inserted whereby the length of each second catheter part is longer than the length of the lumen through which it extends, such that after inserting the second catheter part in the respective lumen the distal end of said second catheter part extends over a predetermined distance outside the distal end of the lumen through said second catheter part.

Taking into account the fact that the lumens through the third catheter part may end each at a different distance of the distal end of the third catheter this embodiment of the catheter provides a very flexible construction for various purposes.

To be able to realize an accurate positioning between the first and second sensor during the insertion of the catheter it is preferred that at the proximal end of the catheter means are present to drive the first catheter part through the lumen in the second catheter part in longitudinal direction such that a predetermined distance between the first and second sensor can be obtained.

In a similar manner an accurate mutual distance between the second and third sensor can be obtained in case that at the proximal end of the catheter means are present for driving the second catheter part through said lumen in the third catheter part in longitudinal direction such that a predetermined distance between the second and third sensor can be obtained.

In general it can be said that for an accurate adjustment of the mutual distances between the sensors it is preferred that near the proximal end of the catheter means are present for driving the (m-1)-th catheter part through the lumen in the m-th catheter part in longitudinal direction such that a predetermined distance between the (m-1)-th sensor and the m-th sensor can be obtained.

For all these moving means it is preferred that they comprise an indication unit providing the user information about the distance over which the respective catheter part is shifted through the respective lumen starting from a known reference position corresponding with a known reference distance between the respective sensors.

In many applications groups of sensors can be used whereby an adjustable distance should be present between the first group and the other group. In this respect an embodiment of the catheter according to the invention has the characteristic that apart from the first sensor other sensors are mounted in or on the, first catheter part and connected to the signal conductors extending through said first catheter part or to further signal conductors.

A further embodiment of the catheter has in this respect the characteristic that apart from the second sensor further sensors are mounted in or on the second catheter part and connected to the signal conductors extending through said second catheter part or to further signal conductors.

In general it is possible to position the various groups of sensors by means of a catheter according to the invention at different locations inside the body, in which respect the catheter has the characteristic that each n-th catheter part may comprise one or more further sensors connected to signal conductors extending through the respective catheter part or to further signal conductors.

In some applications it is possible to follow the insertion and positioning of the sensors for instance by means of X-ray images, magnetic resonance images or similar techniques. In other applications this is not possible and especially for those cases it is preferred that at least one of said catheter parts comprises at least one lumen through which endoscopic light conductors are extending between the distal end of the respective catheter part and suitable coupling means near the proximal end thereof. Through coupling means the endoscopic light conductors can be connected to a television monitor so that the location where the distal end of the inner catheter part is present can be monitored visually. This facilitates the tracing and monitoring of the distal end of the catheter during the insertion into the body and the accurate positioning and adjustment of the sensors of the catheter.

It is remarked that catheters, comprising endoscopic fibres, are known as such, in which respect the attention is drawn to US-5,242,454, US-4,586,491, GB-2,255,281, and FR-2,582,499.

To increase the level of light in the neighbourhood of the distal end of the inner catheter part it may have advantages to install one or more further light conductors in a lumen in that catheter part through which also the endoscopic light conductors are extending, destined for guiding light to the distal end of the catheter. Instead of light within the visible part of the spectrum, also radiation of other wave length in the not visible part of the spectrum can be used.

A catheter according to the invention is especially suited for a catheter system comprising a number of first catheter parts as described in one of the preceding claims and each comprising a different sensor or combination of sensors, as well as a number of second catheter parts as described in one of the preceding claims and each comprising a different sensor or combination of sensors. Therewith the user is provided as it were with a building kit from which depending on the demand a first catheter part with the desired sensors thereon and a second catheter part with the desired sensors thereon and eventually further catheter parts with other desired sensors can be combined into a complete catheter for a specific purpose.

For performing more substantial measurements such a system cannot only comprise a number of first catheter parts and a number of second catheter parts but can comprise in general m-th catheter parts each eventually carrying one or more m-th sensors.

Further embodiments and advantages of the invention will be explained in more detail with reference to the attached drawings.

Figure 1 illustrates a first example of a catheter according to the invention with a first catheter part which is movable inside a second catheter part.

Figure 2 illustrates a second embodiment of a catheter according to the invention with a first and second catheter part, whereby the second catheter part has more than one sensor.

Figure 3 illustrates a third embodiment of a catheter according to the invention with three mutually telescopically movable catheter parts.

Figure 4 illustrates a fourth embodiment of a catheter according to the invention, whereby one catheter part has an inflatable section.

Figure 5 illustrates a fifth embodiment of a catheter according to the invention comprising a telescopic combination of a first, second and third catheter part whereby an additional catheter part extends through a further lumen in the third catheter part.

Figure 6 illustrates schematically a system by means of which various embodiments of a catheter according to the invention can be realized.

One important application of the catheter according to the invention is to obtain a clear indication about the respiratory behaviour of a human being, more specifically an indication about the functioning of the respiratory muscles, from which the diaphragm is the most significant representant. The diaphragm provides 60 to 70% of the respiratory effort.

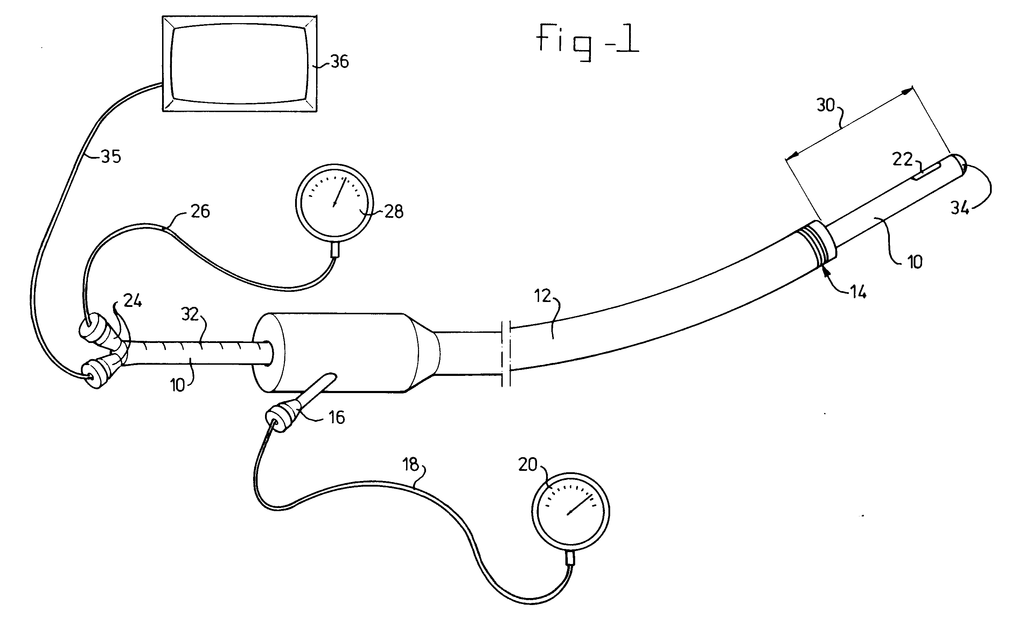

The functional ability of the diaphragm muscles to maintain the respiratory function is one of the important parameters in case the medical staff has to decide wether or not a patient, which is mechanically ventilated, can be weaned from mechanical ventilation. One embodiment of the catheter according to the invention, illustrated in figure 1, is especially destined to provide information about the functioning of the diaphragm muscles and therewith information about the respiratory function of a patient.

The catheter in figure 1 is assembled from a first catheter part 10 and a second catheter part 12. The first catheter part 10 is made of a material having sufficient flexibility and having a sufficient length to enable insertion thereof together with the catheter part 12 at least partly into the esophagus and the stomach. The catheter part 10 carries near its distal end a sensor 22, for instance embodied as an integral pressure sensor. This pressure sensor is through wires (not visible in the figure) which extend through the first catheter part 12 connected to coupling means 24. From the coupling means 24 the pressure sensor signals can be transferred through a cable 26 to an indication unit 28 or to a processor or other suitable means to process these signals.

The second catheter part 12 is also preferably made of a material with a certain flexibility. This second catheter part comprises at least one lumen through which the first catheter part can be inserted. The length of the first part is longer than the length of the lumen through the second part so that the first part can be moved through the second part over such a distance that the distal end thereof can project over a predetermined length outside the distal end of the second part.

The catheter part 12 also comprises a sensor near its distal end, which sensor in this embodiment consists of a series of electromyographic electrodes 14 for measuring local muscular activity by detecting voltage differences. These electrodes 14 are through conductors, extending through the wall of the catheter part 12 or through a further separate lumen from the distal end to the proximal end, connected to a coupling 16. A cable 18 is connected to this coupling and runs to an indication unit 20 or eventually to processor means or similar means for further processing the signals.

As indicated above and as shown in the figure the first catheter part 10 is longer than the lumen through the second catheter part 12 such that the distance 30 between the first sensor 14 and the second sensor 22 can be adjusted by shifting the first catheter part 12 in relation to the second catheter part 10 in longitudinal direction. Preferably the proximal end of the catheter comprises indication means for visualizing the shifting distance to the user. In the very simple embodiment of figure 1 the proximal section of the first catheter part comprises a graduation 32, which is for instance calibrated such that in the situation, whereby the zero line of the graduation 32 coexists with the back edge of the proximal section of the second catheter part 12 the distance between both sensors 14 and 34 equals zero. In that situation the sensor 34 is still present within the lumen through the second catheter part 12 adjacent to the sensor 14. The graduation may have a centimetre/millimetre division. The graduation line which coexists with the back edge of the distal end of the second catheter part indicates the distance between both sensors.

Preferably the first catheter part 12 further comprises a lumen through which an endoscopic fibre bundle (which in figure 1 is not visible inside the catheter) extends from the proximal end until the distal end thereof. Eventually the distal end may comprise a lens 34 for forming an image onto the distal end of the fibre bundle. At the proximal side of the catheter the outwards extending section of the fibre bundle 35 is eventually, through further suited coupling means and image processing circuits connected to a television monitor 36 for visualizing the received image.

As already remarked above, a catheter of the type as illustrated in figure 1 can be used for instance for determining the activity of the diaphragm, which is relevant for determining wether a patient, which is connected to an artificial respiration apparatus, is able to breath independently again, so that the artificial respiration apparatus can be disconnected. For that purpose, the muscular activity of the diaphragm muscles is measured by means of the electromyographic sensor at the location where the diaphragm is attached to the esophagus and furthermore the pressure in the stomach is measured by means of a pressure sensor.

For carrying out such a measurement the catheter according to figure 1 is adjusted such that the graduation 32 is at zero, in other words the distal end of the first catheter 10 is positioned just inside the distal end of the second catheter 12, so that the distance 30 between both sensors 14 and 22 is initially adjusted at zero. The end of the endoscopic bundle, respectively the lens 34, is positioned at the distal end of the second catheter part. In this situation the catheter is inserted into the esophagus, whereby the propagation of the distal end of the catheter can be followed through the images which are transferred through the endoscopic bundle and are visualized onto the screen 36. As soon as exactly the correct position in the esophagus, where the sensor 14 may function optimally, is reached, the propagation of the second catheter part 12 is stopped and now only the first catheter part 10 is moved further. Still the propagation of the distal end thereof can be monitored on the screen 36. As soon as the sensor 22 has reached a suitable place inside the stomach for carrying out pressure measurements, also the longitudinal movement of the first catheter part 10 is stopped and the measurements can be performed.

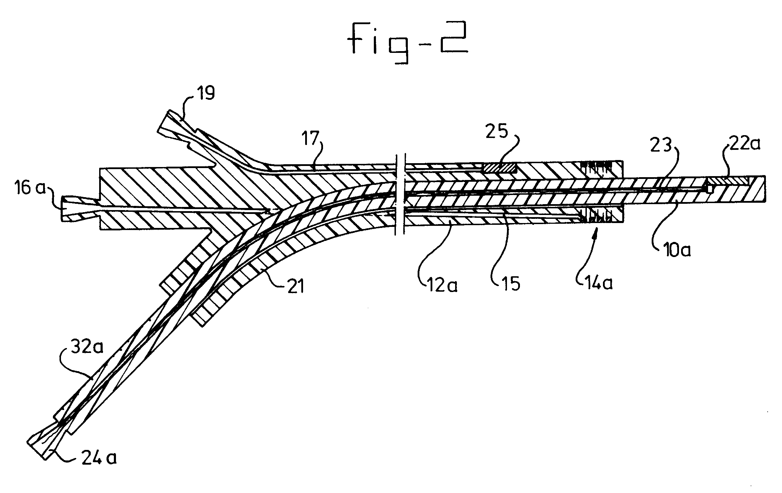

A somewhat modified embodiment of a catheter for this kind of measurements is illustrated in cross section in figure 2.

The catheter in figure 2 is assembled from a first catheter part 10a and a second catheter part 12a. The first catheter part 10a is made of a material with a sufficient flexibility and a sufficient length to enable insertion thereof together with the catheter part 12a depending on the application into the respective bodily cavity. The catheter part 10a carries near its distal end a pressure sensor 22a. This pressure sensor is connected to coupling means 24 through wires 23 which extend through the first catheter part 12a. From the coupling means 24a the pressure sensor signals can be transferred to a not illustrated indication unit or a processor or other suitable means to process these signals.

The second catheter part 12a is also preferably made of a material with a certain flexibility. The second catheter part comprises at least one lumen through which the first catheter part can be inserted. The length of the first part is longer than the length of the lumen through the second part so that the first part can be shifted through said second part over such a distance that the distal end thereof can project over a predetermined distance outside the distal end of the second part.

In this embodiment the catheter part 12a comprises both a pressure sensor 25 for measuring the pressure inside the esophagus as well as a series of electromyographic electrodes 14a for locally measuring the muscular activity by means of monitoring voltage differences. The electrodes 14a are connected through conductors 15 extending through the wall of the catheter part 12a or through a separate further lumen from the distal end to the proximal end in connection with a coupling 16. A cable, which is not illustrated in the figure, can be connected to this coupling 16 and may lead to an indication unit or eventually to processing means, etc., for further processing the signals. Through conductors 17, which extend from the distal end through the wall of the catheter part 12a or through a further separate lumen, the pressure sensor 25 is connected to a coupling 19 which in this case is attached to a sidewards protuberance of the catheter part 12a near the distal end thereof. A non-illustrated cable may connect this coupling 19 to an indication unit or eventually to further processor means for further processing the pressure sensor signals.

It is remarked that in a situation wherein the electromyographic sensors 14a are present in a larger space, such as in a cardiovascular application, and do not contact muscular tissue, the sensors can be used for volume measurements. This, however, is known to the expert in this field.

As is illustrated in the figure in this embodiment the second catheter part is inserted into the lumen through the first catheter part via a sidewards protuberance 21 of the first catheter part 12a. The first catheter part 10a is again longer than the lumen through the second catheter part 12a such that the distance 30a between the first pressure sensor 14a and the electromyographic sensor 22a can be adjusted by shifting the first catheter part 12a in relation to the second catheter part 10a in longitudinal direction. Also in this case a graduation 32a is used for determining the displacement of the proximal end of the first catheter part.

Also the catheter of the type as illustrated in figure 2 may be used for instance for determining the activity of the diaphragm, in a similar manner as the catheter in figure 1. For carrying out such a measurement the catheter according to figure 2 is adjusted such that the graduation 32 is at zero, in other words the distal end of the first catheter 10a is positioned just inside the distal end of the second catheter 12a, so that the distance between both sensors 14a and 22a is initially adjusted at zero. In this configuration the catheter is inserted into the esophagus, whereby the propagation of the distal end of the catheter can be followed for instance by means of a suitable X-ray apparatus as is known to the expert in this field. As soon as exactly the correct position in the esophagus, where the sensor 14a may function optimally, is reached, the propagation of the second catheter part 12a is stopped and now only the first catheter part 10a is moved further. Still the propagation of the distal end thereof can be monitored using the X-ray images. As soon as the sensor 22 has reached a suitable location inside the stomach for carrying out pressure measurements, also the longitudinal movement of the first catheter part 10a is stopped and the measurements can be performed. The advantage of this second embodiment is that thanks to the presence of the second pressure sensor 25 which in the end situation is positioned inside the esophagus, also the pressure in the esophagus can be measured simultaneously with the pressure in the stomach.

In this second embodiment the catheter does not comprise an endoscopic bundle and for determining the position use is made of X-ray images. The advantage thereof is that the diameter of the first catheter part is almost exclusively determined by the dimensions of the first sensor 22a. In a preferred embodiment of the catheter according to the invention the diameter of the first catheter part is not more than 1 millimetre. Such an embodiment is specially suited for performing cardiovascular measurements, specially measurements for determining the extent of a partial blockage or narrowing of a blood vessel. The catheter is thereby manoeuvred and positioned such that the electromyographic array of sensors 14a is located at the narrow spot whereby the one pressure sensor 22a is located at the one side of the narrow spot and the other pressure sensor 25 is located at the other side of the narrow spot.

Under circumstances it is sufficient to measure only the pressure inside the stomach and inside the esophagus to obtain sufficient information. In that case the electromyographic sensor 14a (together with the coprresponding conductors 15 and the connector 16) can be left out so that the catheter only comprises a first pressure sensor 22 a on the first catheter part 10a and a second pressure sensor 25 on the second catheter part 12a, the mutual distance between both sensors being adjustable by shifting the first catheter part through the second catheter part.

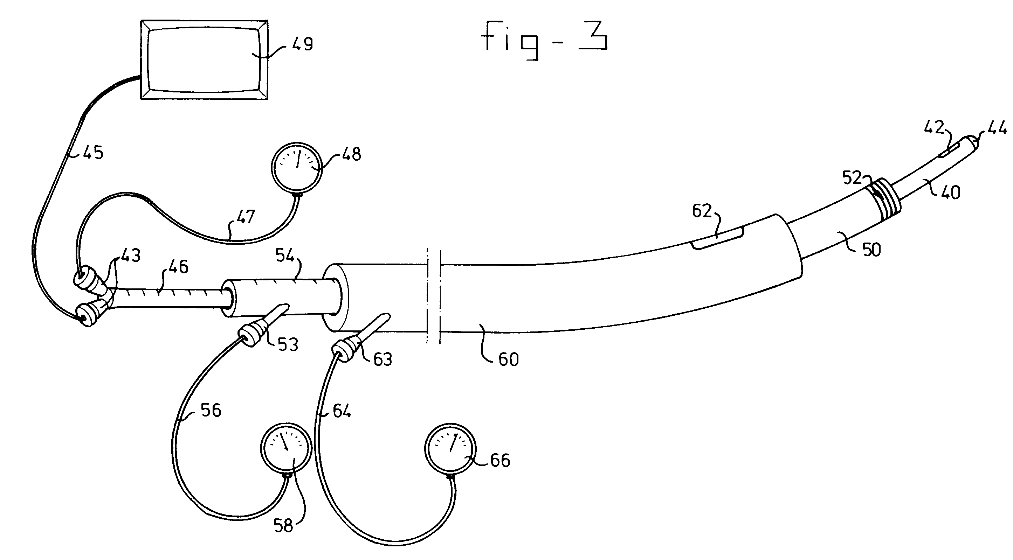

A third embodiment of a catheter according to the invention which can be used both for applications in the gatrointestinal tract, in the urology area as well as for cardiovascular applications, is very schematically illustrated in figure 3. This embodiment of the catheter comprises a first catheter part 40 carrying at the distal end thereof a pressure sensor 42. This pressure sensor 42 is connected through a signal conductor which extends in the wall of the catheter 40 or through a separate lumen therein to coupling means 43 from which a signal wire 17 extends to an indication instrument 48 or to another circuit or processor for further processing the sensor signals. An endoscopic bundle extends through a lumen in the first catheter part 40, said bundle ending at the distal side in a lens 44 whereas at the proximal side this bundle, of which the visual part is referred by 45, is connected to an image processing and indication unit 49. This endoscopic bundle can be removed in case for instance X-ray images are used for positioning purposes.

The catheter furthermore comprises a second catheter part 50 of which the length is shorter than the lumen through the first catheter part 40. This second catheter part 50 comprises at its distal end electromyographic electrodes 52 which through signal conductors are connected to a coupling 53. A signal cable 56 extends during operation from this coupling 53 to a measurement instrument 58 or to another data processing apparatus. Finally, in this embodiment the catheter comprises a third catheter part 60 which near its distal end comprises a pressure sensor 62. This pressure sensor 62 is connected through signal conductors extending through the wall of the catheter part 60 or through a separate lumen to the coupling 63. During operation a signal cable 64 extends from the coupling 63 to a measurement apparatus or eventually to other data processing means.

On the proximal end section of the first catheter part 40 a graduation 46 is applied by means of which, as explained above, the distance can be adjusted between the sensor 42 and the sensor 52. On the proximal section of the second catheter part 50 a graduation 54 is applied by means of which in a similar manner the distance between the sensor 52 and the sensor 62 can be adjusted.

This embodiment of the catheter for instance can be used for performing measurements in the bladder and in the urethra, for instance with incontinence patients. Just as in the last described embodiment also in this embodiment one starts with an initial situation in which the distal ends of the three catheter parts are matching, in other words the distances between the various sensors are adjusted at zero and by means of the display 49 the propagation of the distal end of the first catheter part can be followed on the screen. The catheter is inserted in the urethra until the pressure sensor 62 has reached its desired location. From that moment on the third catheter part 60 is hold steady and only the first and second catheter part 40, 50 are moved further until the sensor 52 has reached its desired location to measure the muscular activity, which is amongst other responsible for pinching off and closing the urethra in a suitable manner. The second catheter part 50 is hold steady thereafter and only the first catheter part 40 is moved further until the sensor 42 has reached a suitable location within the urethra or inside the bladder to measure the internal local pressure.

It will be clear that the embodiment illustrated in figure 3 can be used also for the above described application in the esophagus and stomach for determining wether or not a patient, which is mechanically ventilated, can be weaned from mechanical ventilation.

Furthermore this embodiment as very suited for application in the gastrointestinal tract. As remarked above the endoscopic bundle 45 and the lens 44 can be omitted in case other means are used for positioning. The then non-used central lumen through the first catheter part can be used for instance for feeding a nutrient feeding solution to the stomach or to the upper portion of the duodenum. A similar application in this area is described in the already mentioned US 4,921,481.

In this respect the attention is drawn to the fact that in many cases patients which are mechanically ventillated are are also coonected to a feeding catheter for delivering nutrients to either the stomach or to the upper portion of the duodenum. A catheter of the type illustrated in figure 3, in which the endoscopic bundle is removed and replaced by an empty lumen, can be used for both purposes. In the specific case that the nutrient solution has to be delivered direcly to the upper portion of the duodenum the first catheter part could carry two pressure sensors, one near the distal tip for sensing the pressure in the duodenum and another sensor at some distance thereof to sense the pressure inside the stomach.

In case the nutrient solution has to be delivered to the stomach of a petient which is mechanically ventillated the embodiment illustrated in figure 3 can be applied as such. For measuring the respiratory function of the patient the first catheter section carrying the first pressure sensor is inserted in the second catheter part until the first sensor is located inside the stomach. For feeding a nutrient solution the first catheter part is completely withdrawn and the free lumen through the second catheter part is then used for feeding purposes.

The catheter according to figure 3 can also be used for cardiovascular applications especially for measuring the pressure and volume in a blood vessel which is more or less blocked. As is explained with reference to figure 2 the catheter is thereby manoeuvred and positioned such that the electromyographic array of sensors 52 is located at the narrow spot, whereby the one pressure sensor 42 is present at the one side of the narrow spot and the other pressure sensor 62 is present at the other side of the narrow spot.

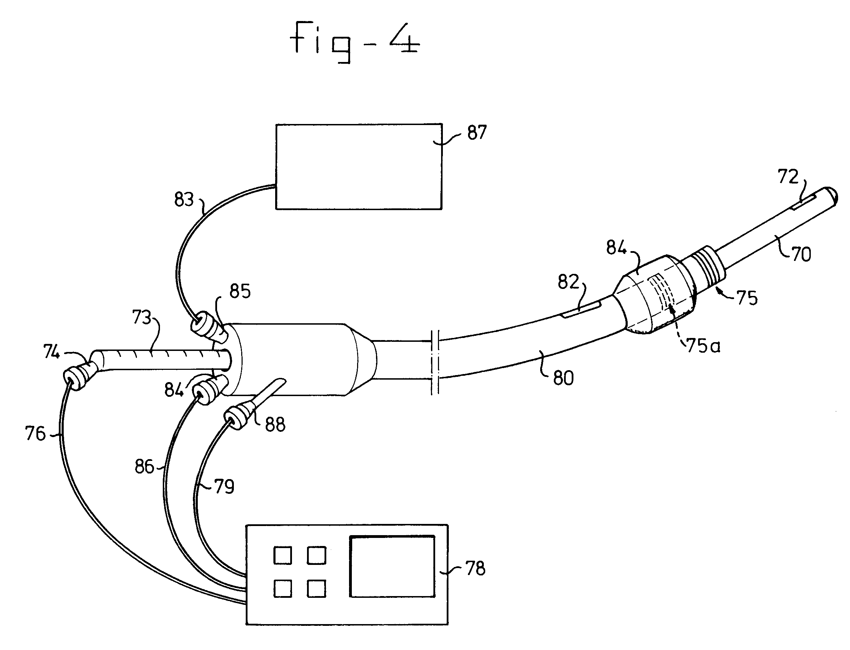

Figure 4 illustrates a fourth embodiment of a catheter according to the invention. This embodiment comprises a first catheter part 70 with a pressure sensor 72 near the distal end thereof. Furthermore this catheter comprises a second catheter part 80 with an array of electromyographic electrodes 75 near the distal end thereof and a pressure sensor 82 at some distance of the distal end and between the array 75 and the pressure sensor 82 an inflatable balloon section 84. The pressure sensor 72 is connected to a connector 74 at the proximal side through a signal conduit which is embedded in the first catheter part or extends through a lumen therein. During operation the coupling 74 is connected through a cable 76 to a data processing processor 78 for making the sensor signals for instance visible on a display screen. In the same manner the pressure sensor 82 is through a conduit through the second catheter part 80 connected to a coupling 84 at the proximal end thereof. During operation a signal cable 86 runs to a processor 78. The array of sensors 75 is through further signal conduits connected to a coupling 88, from where a connection is made through a conduit 79 to the signal processor 78. The proximal end of the first catheter part carries again a graduation 73 for indicating the distance between the sensors 72 and 82.

The element, which is new in respect to the embodiments illustrated in figures 1 and 2, is the inflatable balloon 84. Through the second catheter part 80 extends a separate lumen from a coupling 85 at the proximal end until an opening in the wall of the catheter such, that in case from the coupling 85 a fluidum is pressed into this lumen the balloon 84 will be inflated. By sucking off the fluidum the balloon 84 will be deflated and is brought preferably in such a shape that the outside diameter of the second catheter part at the location of the balloon 84 at least approximately corresponds with the outside diameter of the other sections of the second catheter part. During operation a fluidum pump, for instance a normal air pressure pump or a fluid pump 87 can be connected through a conduit 83 to the coupling 85 to inflate or deflate the balloon 84.

A catheter of the embodiment, illustrated in figure 4, can be for instance used for applying a blockage at a predetermined location by means of the balloon 84, for instance in the esophagus, in a predetermined blood vessel, in the gastrointestinal channel, etc., and for successively measuring the pressure at both sides of this artificial blockage. Especially such a catheter is suitable for performing a dilatation treatment in a blood vessel. By means of the sensors the effect of this treatment can be measured directly.

An important application area of such catheters is the determination of the diameter of a blood vessel, the urethra, etc. For these applications it is especially preferred that the balloon is embodied such that this balloon has a circular outer surface in the inflated situation. If ring-shaped conductance electrodes 75a are attached into the balloon in or on the second catheter part as schematically illustrated in the respective section of the balloon 84, then it is possible, when a suitable fluidum is applied for inflating the balloon, to measure the cross section of the urethra, the blood vessel, etc., accurately on the ground of the conductance value signals.

Furthermore a catheter of the type illustrated in figure 4 can be used as Intra Arterial Blood Pump. For details about the functioning of such a pump reference is made to for instance US 5,413,549.

In many of the mentioned applications it is preferred that there are also electromyographic sensors present within the balloon section 84 to be able to measure the cross section of the balloon.

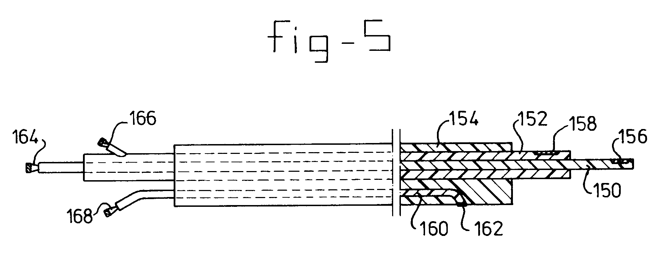

Another embodiment is illustrated in figure 5, on the left hand side in exterior view and on the right hand side in cross section. The therein shown catheter comprises a telescopic combination of three catheter parts 150, 152, and 154. The part 150 comprises a sensor 156 and the part 152 comprises a sensor 158. The part 150 may shift through a lumen in part 152 and the part 152 may shift through a lumen in part 154. In the catheter part 154 a further lumen is present through which a further catheter part 160 with a sensor 162 at its distal end can be shifted.

The sensor 156 is through a conduit, which is for the sake of clearness not illustrated in the figure, connected to the connector 164, the sensor 158 is through an also not shown conduit connected to the connector 166 and the sensor 160 is through a not shown conduit connected to the connector 168.

With this embodiment of the catheter it is possible, after positioning of the various catheter parts and after performing the desired measurements, to withdraw for instance the catheter part 160 and to replace this part by another catheter part with another type of sensor so that another combination of measuring signals can be obtained without the necessity to remove the other catheter parts and without disturbing the environment used for the first measurements.

Furthermore, this catheter offers the possibility to remove the catheter part 156 or the catheter part 160 while the other sensors are still functioning, whereafter the then free respective lumen can be used for applying a nutrient solution, medicines, narcotics, or other substances.

Although in the above described embodiments only pressure sensors and electromyographic sensors are used, it will be clear that many other types of sensors can be used dependent on the desired application. One may think of temperature sensors, oxygen concentration sensors, pH sensors, and integrated flow detection circuits.

To provide the user the possibility to assemble at wish catheters for various applications, it is preferred to provide the assembling parts of the catheter in the form of a catheter system comprising a number of first catheter parts, a number of second catheter parts and eventually a number of further catheter parts, each comprising different sensors.

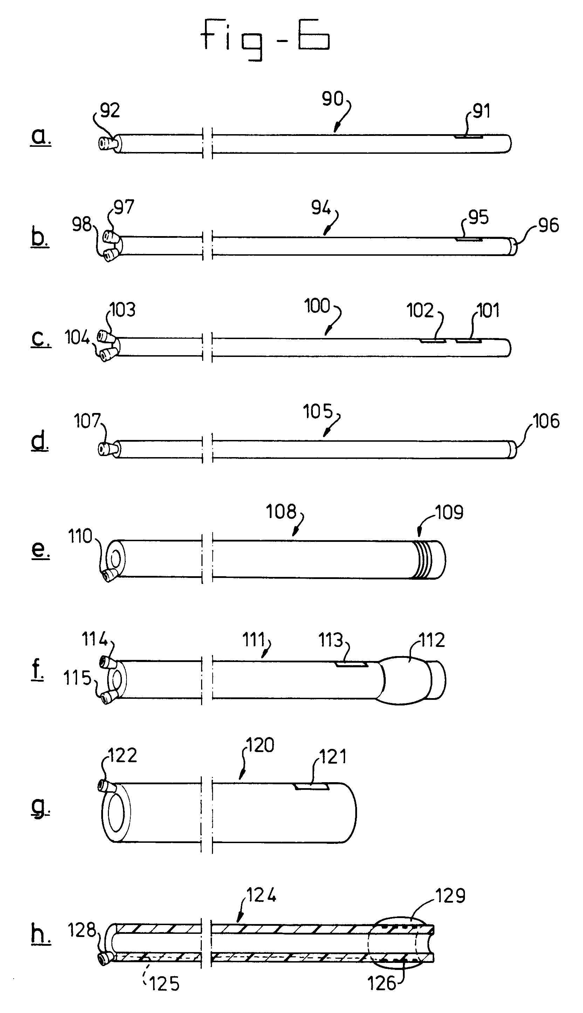

An example of such a catheter system is schematically illustrated in figure 6. In figure 6 at a) a first catheter part 90 is shown comprising a pressure sensor 91 at the distal end and a coupling 92 at the proximal end. Between the sensor 91 and the coupling 92 extends a signal conductor which is not visible in the figure. At b) a first catheter part 94 is shown comprising a pressure sensor 95 at the distal end and a corresponding coupling 97 at the proximal end. Furthermore, this catheter part comprises endoscopic conductors from a lens 96 at the distal end to an additional coupling 98 at the proximal end. At c) a first catheter part 100 is shown comprising both a pressure sensor 101 as well as a temperature sensor 102. Through signal conductors which are not visible in the figure, the pressure sensor 101 is connected to the coupling 103 whereas the temperature sensor 102 is connected to the coupling 104, both couplings situated near the proximal end. At d) a first catheter part 105 is shown comprising a blood glucose sensor 106 near the distal end which through a signal conduit is connected to a coupling 107 near the proximal end. At e) a second catheter part 108 is shown comprising electromyographic electrodes 109 near the distal end which through signal conductors extending through said catheter part are connected to a coupling 110 near the proximal end. At f) a second catheter part 111 is shown comprising a pressure sensor 113 which through a signal conduit is connected to the couplings means 114 and comprises a balloon section 112 between this pressure sensor 113 and the distal end of the catheter part, which balloon section through a lumen is connected to a coupling 115 at the proximal end. At g) a third catheter part 120 is shown comprising a pressure sensor 121 which through a signal wire is connected to coupling means 122. At h) a second catheter part 124 is shown in cross sections, comprising an inflatable balloon section 129, inside of which electromyographic sensors 126 are positioned. These sensors 126 are through conduit 125 connected to coupling 128.

It will be clear from these figures that for instance the embodiment illustrated in figure 1 can be assembled making use of the components b) and e) from the system which is shown in figure 6. The catheter shown in figure 2 can be assembled by using the components b), e), and g). The catheter in figure 3 can be assembled with the components b) and f). For other applications the expert in this field can assemble catheters at wish from this kit suited for the application.

## Claims

1. Catheter for accurately positioning a number of sensors at various locations inside a patient's body to measure sensor dependent parameters at said various predetermined locations, which catheter comprises at least a first sensor near the distal end of the catheter, and at least a second sensor at a distance of the first sensor, first and second coupling means near the proximal end of the catheter for transferring the signals of said first and second sensors, and first and second signal conductors between the respective first and second sensors and coupling means, characterized in that the catheter comprises:
- a first elongated catheter part near the distal end of which the first sensor is mounted and through which the first signal conductors extend to the first coupling means,
- a second elongated catheter part near the distal end of which the second sensor is mounted and through which the second signal conductors extend to the second coupling means, which second catheter part comprises at least one lumen through which the first catheter part, which is longer than said lumen through the second catheter part, can be inserted from the proximal end thereof, such that the distal end of the first catheter part projects over a predetermined distance outside the distal end of the second catheter part.

2. Catheter according to claim 1, characterized in that the catheter comprises:
- a number of first catheter parts each comprising a sensor near the distal end of the respective first catheter part and each comprising signal conductors between said sensor and coupling means near the proximal end, and
- that the second catheter part comprises a number of lumen, corresponding with the number of first catheter parts, through each of which one of said first catheter parts can be inserted, whereby the length of each of said first catheter parts is longer than the length of the lumen through which it is inserted such, that after inserting a first catheter part in the respective lumen the distal end of said first catheter part projects over a predetermined distance outside the distal end of the lumen through said second catheter part.

3. Catheter according to claim 1, characterized in that the length of the first catheter part respectively each first catheter part is at least equal to the length of the respective lumen through said second catheter part increased by the maximum distance over which the distal end of the first catheter part has to project outside the distal end of the lumen through said second catheter part.

4. Catheter according to claim 1, 2, or 3, characterized in that the lumen respectively at least one of the lumen through said first catheter part ends at a predetermined distance of the distal end of the first catheter part.

5. Catheter according to one of the preceding claims, characterized in that the catheter comprises:
- a third elongated catheter part, which third catheter part comprises at least one lumen through which the second catheter part, which is longer than said lumen through the third catheter part, can be inserted from the proximal end thereof, such that the distal end of the second catheter part projects over a predetermined distance outside the distal end of said lumen through said third catheter part.

6. Catheter according to claim 5, characterized in that near the distal end of the third catheter part a third sensor is mounted and that third signal conductors are extending through said third catheter part to third coupling means at the proximal end of the catheter.

7. Catheter according to claim 4 or 5, characterized in that the catheter comprises n elongated catheter parts (n equal to or larger than 4) whereby each m-th elongated catheter part (m at least equal to 2 but at the most equal to n) comprises at least one lumen through which the (m-1)-th catheter part, which is longer than the lumen through the m-th catheter part can be inserted from the proximal end thereof such that the distal end of the (m-1)-th catheter part projects over a predetermined distance outside the distal end of the lumen through said m-th catheter part.

8. Catheter according to claim 7, characterized in that near the distal end of m-th catheter part an m-th sensor is mounted and m-th signal conductors are extending through said m-th catheter part to m-th coupling means at the proximal end of the catheter.

9. Catheter according to claim 1, characterized in that the catheter comprises:
- a number of first catheter parts, each comprising a sensor near the distal end of the respective first catheter part and each comprising signal conductors between said sensor and coupling means near the proximal end, and
- a number of second catheter parts, each comprising a lumen through which a first catheter part can be inserted, whereby the length of said first catheter part is longer than the length of the lumen through which it extends such that after inserting the first catheter part in the respective lumen the distal end of the first catheter part projects over a predetermined distance outside the distal end of the lumen through said second catheter part,
- a third catheter part, comprising a number of lumen, corresponding to the number of second catheter parts, through each of which one of said second catheter parts can be inserted whereby the length of each second catheter part is longer than the length of the lumen through which it extends, such that after inserting the second catheter part in the respective lumen the distal end of said second catheter part extends over a predetermined distance outside the distal end of the lumen through said second catheter part.

10. Catheter according to one of the preceding claims, characterized in that at the proximal end of the catheter means are present to move the first catheter part through the lumen in the second catheter part in longitudinal direction such that a predetermined distance between the first and second sensor can be obtained.

11. Catheter according to one of the preceding claims 5 until 10, characterized in that at the proximal end of the catheter means are present for moving the second catheter part through said lumen in the third catheter part in longitudinal direction such that a predetermined distance between the second and third sensor can be obtained.

12. Catheter according to one of the preceding claims 7 until 11, characterized in that near the proximal end of the catheter means are present for moving the (m-1)-th catheter part through the lumen in the m-th catheter part in longitudinal direction such that a predetermined distance between the (m-1)-th sensor and the m-th sensor can be obtained.

13. Catheter according to one of the preceding claims 8, 9, 10, or 11, characterized in that said moving means comprise an indication unit providing the user information about the distance over which the respective catheter part is moved through the respective lumen starting from a known reference position corresponding with a known reference distance between the respective sensors.

14. Catheter according to one of the preceding claims, characterized in that apart from the first sensor other sensors are mounted in or on the first catheter part and are connected to the signal conductors extending through said first catheter part or to further signal conductors.

15. Catheter according to one of the preceding claims, characterized in that apart from the second sensor further sensors are mounted in or on the second catheter part and are connected to the signal conductors extending through said second catheter part or to with further signal conductors.

16. Catheter according to one of the preceding claims, characterized in that each n-th catheter part may comprise one or more further sensors connected to the signal conductors extending through the respective catheter part or to further signal conductors.

17. Catheter according to one of the preceding claims, characterized in that at least one of said catheter parts comprises at least one lumen through which endoscopic light conductors are extending between the distal end of the respective catheter part and specially determined coupling means near the proximal end thereof.

18. Catheter according to claim 17, characterized in that at least the first catheter part comprises a lumen through which endoscopic light conductors are extending.

19. Catheter according to claim 18, characterized in that one or more further light conductors are inserted in a lumen through the first catheter part and are destined for conducting light to the distal end of the catheter.

20. Catheter according to one of the preceding claims, characterized in that the second catheter part or one of the second catheter parts comprises a wall section which can be blown up into a balloon shape.

21. Catheter according to one of the preceding claims, characterized in that at least one of said catheter parts comprises at least one lumen extending from the proximal end of the respective catheter part through the opening in the wall of the catheter part at a distance of the distal end thereof, through which lumen an elongated element comprising a sensor at the distal end thereof can be inserted.

22. Catheter according to one of the preceding claims, characterized in that the diameter of the first catheter respectively each first catheter including the sensor mounted therein or thereon is at the most equal to one millimetre.

23. Catheter system comprising a number of first catheter parts as described in one of the preceding claims and each comprising a different sensor or combination of sensors, as well as a number of second catheter parts as described in one of the preceding claims and each comprising a different sensor or combination of sensors.

24. Catheter system according to claim 23, characterized in that the system comprises a number of third catheter parts as described in one of the preceding claims and each comprising a different sensor or combination of sensors.
